# EUROPEAN PATENT APPLICATION

(11) **EP 4 098 229 A1**
(43) Date of publication of application: **07.12.2022**
(21) Application number: 22177017.5
(22) Date of filing: 02.06.2022
(51) Int. Cl.: A61F 2/64, A61F 2/74

(54) **KNEE JOINT, PROSTHETIC LEG, AND ROTATIONAL RESISTANCE MODULE**

(30) Priority: 03.06.2021 JP 2021093611
(71) Applicant: Nabtesco Corporation, Tokyo 102-0093 (JP)
(72) Inventor: Okuda, Masahiko, Tokyo, 102-0093 (JP)
(74) Representative: Grünecker Patent- und Rechtsanwälte PartG mbB

(57) **Abstract**

A knee joint (20) includes: a thigh connection portion (22) that is provided on a thigh portion side; a lower leg portion (21) that is connected to the thigh connection portion (22) and rotatably provided around a knee shaft; a knee angle restrictor (24) structured to restrict a relative rotation of the thigh connection portion (22) and the lower leg portion (21) such that a knee angle θ formed by the thigh connection portion (22) with respect to the lower leg portion (21) does not exceed a predetermined lock angle θ_{L}; and an accommodation space that accommodates a rotational resistor applying resistance to the rotation around the knee shaft in a case where the knee angle θ is equal to or less than the lock angle θ_{L}.

## Description

The present invention relates to a knee joint.

In JP H09-75382 A, a knee joint or a prosthetic leg worn by a person who has lost a leg due to injury or illness is disclosed, and a knee angle formed by a thigh portion and a lower leg portion is locked so as not to exceed a predetermined lock angle. In a locked state, it is possible to prevent knee bending of which an angle exceeds an unintended lock angle of a wearer of the knee joint, and in an unlocked state, it is possible to perform a sitting operation of largely bending the knee with exceeding the lock angle.

In the locked state, the knee angle is limited not to exceed the lock angle, but in a state in which the knee angle is equal to or less than the lock angle, the knee joint can be freely bent. For this reason, for example, when the sudden knee bending having a lock angle occurs from a state in which the knee angle at which the thigh portion and the lower leg portion are on a straight line is zero degrees, there is a possibility that balance of a body of the wearer of the knee joint is lost and the wearer falls down. Furthermore, even when the wearer does not fall down, there is a possibility that the wearer of the knee joint feels greatly uneasy due to the sudden knee bending having the lock angle.

The present invention has been made in view of such a situation, and an object of the present invention is to provide a knee joint capable of preventing a sudden knee bending.

In order to solve the above problems, according to an aspect of the present invention, there is provided a knee joint including: a thigh connection portion that is provided on a thigh portion side; a lower leg portion that is connected to the thigh connection portion and rotatably provided around a knee shaft; a knee angle restrictor structured to restrict a relative rotation of the thigh connection portion and the lower leg portion such that a knee angle formed by the thigh connection portion with respect to the lower leg portion does not exceed a predetermined lock angle; and a rotational resistor structured to apply resistance to the rotation in a case where the knee angle is equal to or less than the lock angle.

According to this aspect, the resistance applied by the rotational resistor can prevent a sudden knee bending.

According to another aspect of the present invention, there is provided a prosthetic leg. The prosthetic leg includes: a thigh portion; a thigh connection portion that is provided on the thigh portion side; a lower leg portion that is connected to the thigh connection portion and rotatably provided around a knee shaft; a knee angle restrictor structured to restrict a relative rotation of the thigh connection portion and the lower leg portion such that a knee angle formed by the thigh connection portion with respect to the lower leg portion does not exceed a predetermined lock angle; and a rotational resistor structured to apply resistance to the rotation in a case where the knee angle is equal to or less than the lock angle.

According to still another aspect of the present invention, there is provided a rotational resistance module. The rotational resistance module is capable of being attached to and detached from a knee joint including: a thigh connection portion that is provided on a thigh portion side; a lower leg portion that is connected to the thigh connection portion and rotatably provided around a knee shaft; and a knee angle restrictor structured to restrict a relative rotation of the thigh connection portion and the lower leg portion such that a knee angle formed by the thigh connection portion with respect to the lower leg portion does not exceed a predetermined lock angle, and resistance is applied to the rotation in a case where the knee angle is equal to or less than the lock angle.

According to still another aspect of the present invention, there is provided a knee joint. The knee joint includes: a thigh connection portion that is provided on a thigh portion side; a lower leg portion that is connected to the thigh connection portion and rotatably provided around a knee shaft; a link mechanism in which one ends of a first link having the other end provided on the thigh connection portion side and a second link having the other end provided on the lower leg portion side are rotatably connected to each other; and a knee angle restrictor structured to restricts a relative rotation of the thigh connection portion and the lower leg portion such that a knee angle formed by the thigh connection portion with respect to the lower leg portion does not exceed a predetermined lock angle, the knee angle restrictor including an elastic member with which at least one of the first link or the second link comes into contact when at least the knee angle is the lock angle.

Note that an arbitrary combination of the above constituent elements and expressions of the present invention made by using a device, a system, a recording medium, a computer program, and the like are also effective as the aspect of the present invention.
Fig. 1 illustrates a schematic configuration of an electronically controlled passive type of knee joint and prosthetic leg;
Fig. 2 illustrates a schematic configuration of an electronically controlled passive type of knee joint and prosthetic leg;
Fig. 3 schematically illustrates transitions of a knee angle θ, a thigh angle Ψ, and a shin angle Φ according to a walking phase of a wearer of a prosthetic leg;
Fig. 4 is a diagram illustrating a cylinder and a drive mechanism;
Figs. 5A and 5B are diagrams illustrating a flow of oil at a time of bending and stretching motion of a knee joint;
Fig. 6 illustrates a first configuration example of a mechanical passive type of knee joint;
Figs. 7A and 7B illustrate a bending and stretching motion of a mechanical passive type of knee joint of the first configuration example;
Fig. 8 illustrates a second configuration example of a mechanical passive type of knee joint;
Figs. 9A and 9B illustrate a bending and stretching motion of a mechanical passive type of knee joint of the second configuration example;
Figs. 10A to 10D illustrate a rotational operation of a knee shaft in an unlocked state;
Fig. 11 illustrates a third configuration example of a mechanical passive type of knee joint;
Figs. 12A and 12B illustrate a bending and stretching motion of a mechanical passive type of knee joint of the third configuration example;
Fig. 13 illustrates different lock angles according to a position of an elastic member; and
Fig. 14 illustrates a rotational operation of a thigh connection portion in an unlocked state.

The invention will now be described by reference to the preferred embodiments. This does not intend to limit the scope of the present invention, but to exemplify the invention.

There are various types of prosthetic legs, and the prosthetic legs are roughly classified into an active type and a passive type. The passive type is further divided into an electronically controlled type and a mechanical type. In the active type, a joint such as a knee or an ankle is actively bent and stretched by an actuator such as a motor. In the electronically controlled passive type, the resistance of the joint is controlled by an electronically controlled hydraulic cylinder or the like according to the bending and stretching motion of the wearer during walking. In the mechanical passive type, locking of the knee angle or the like is performed by a mechanical mechanism as in JP H09-75382 A. Although the present invention is applicable to any type of the prosthetic legs, first, an electronically controlled passive type of prosthetic leg will be described.

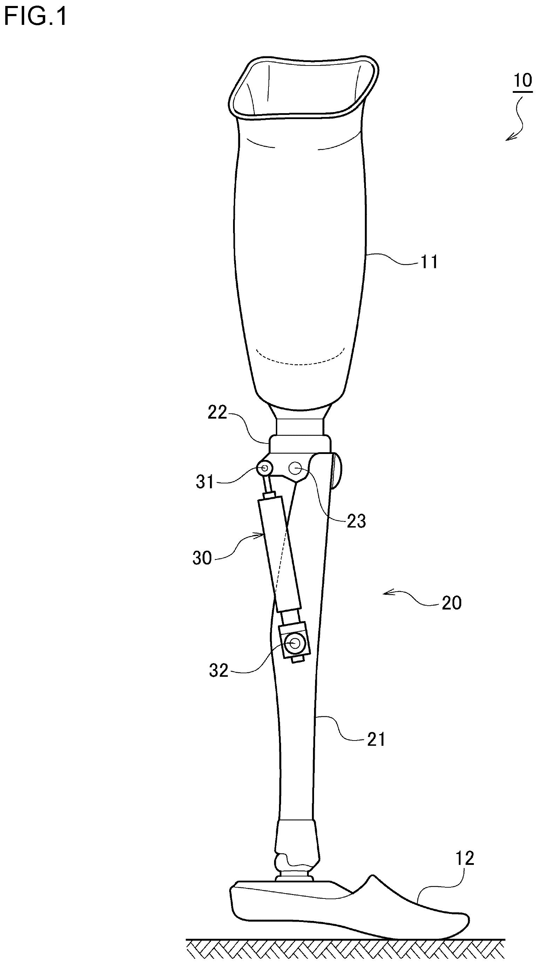

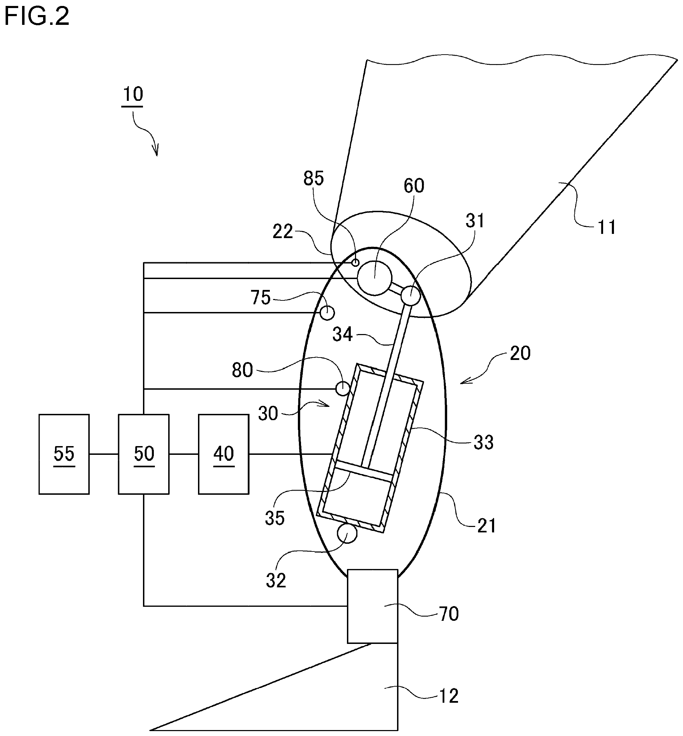

Figs. 1 and 2 illustrate a schematic configuration of an electronically controlled passive type of knee joint 20 and prosthetic leg 10. The prosthetic leg 10 includes a plastic socket 11 as a thigh portion, a thigh connection portion 22 to which the socket 11 is connected, a lower leg portion 21 connected to the thigh connection portion 22 and rotatably provided around a knee shaft 23, and a foot portion 12 connected to a lower end of the lower leg portion 21. The thigh connection portion 22 to which the socket 11 is connected and the lower leg portion 21 bend and stretch the knee joint 20 corresponding to the knee joint by a relative rotation around the knee shaft 23 perpendicular to the paper plane of Fig. 1, the knee shaft 23 being provided at a connection portion of the thigh connection portion 22 and the lower leg portion 21. Furthermore, the lower leg portion 21 and the foot portion 12 are connected via an elastic member, and relative orientations of the lower leg portion 21 and the foot portion 12 when a load from the ground is small at the time when the leg does not come into contact with the ground or the leg stands upright are kept constant by elasticity. Furthermore, when the load from the ground is great at the time of walking, the elastic member is elastically deformed to generate a propulsive force for kicking the ground.

The knee joint 20 includes the lower leg portion 21 formed by a high-strength frame, the thigh connection portion 22 connected to the socket 11 as the thigh portion and connected to the lower leg portion 21 so as to be rotatable around the knee shaft 23, a cylinder 30 that restricts or assists a rotational operation around the knee shaft 23, that is, a bending and stretching motion of the knee joint 20, and a drive mechanism 40 that drives the cylinder 30.

The expansion and contraction amount of the cylinder 30 and the knee angle that is a rotation angle of the knee joint 20 around the knee shaft 23 are in one-to-one correspondence, and a knee angle sensor 60 as a knee angle detection unit, which measures the expansion and contraction amount of the cylinder 30 and detects the knee angle of the knee joint 20, is provided near the cylinder 30 and the thigh connection portion 22. The knee angle detected by the knee angle sensor 60 is used by a control unit 50. The knee angle sensor 60 can be configured by any sensor capable of measuring the expansion and contraction amount of the cylinder 30, but can be configured by, for example, a Hall element capable of detecting a position of a magnet embedded in a piston rod 34 that moves with the expansion and contraction of the cylinder 30. The knee angle is an angle formed by an axis of the socket 11 as the thigh portion and an axis of the lower leg portion 21. For example, as illustrated in Fig. 1, in a case where the wearer of the prosthetic leg 10 stands, and the axis of the socket 11 and the axis of the lower leg portion 21 are on a straight line, the knee angle is zero degrees. Furthermore, in a case where the wearer of the prosthetic leg 10 sits and the axis of the socket 11 changes in a horizontal direction while maintaining the axis of the lower leg portion 21 in a vertical direction in Fig. 1, the knee angle becomes 90 degrees.

An inertial sensor 75 provided in the lower leg portion 21 measures speed (angular velocity) and/or acceleration (angular acceleration) in a translational direction and/or a rotational direction of three axes for the motion of the lower leg portion 21, and detects the orientation and motion of the lower leg portion 21. Among the orientations of the lower leg portion 21 detected by the inertial sensor 75, the shin angle, which is an inclination angle formed by the axis of the lower leg portion 21 with respect to the vertical line, is particularly useful in controlling the knee joint 20. The inertial sensor 75 can be installed at any place of the lower leg portion 21, and, for example, is mounted, together with the control unit 50, on a control board installed on the outer periphery of the cylinder 30.

The thigh angle, which is an inclination angle formed by the axis of the socket 11 as the thigh portion with respect to the vertical line, can be calculated from the knee angle acquired by the knee angle sensor 60 and the shin angle acquired by the inertial sensor 75. That is, since the shin angle is the inclination of the lower leg portion 21 with respect to the vertical line and the knee angle is the inclination of the axis of the socket 11 with respect to the axis of the lower leg portion 21, it is possible to calculate the thigh angle which is the inclination of the axis of the socket 11 with respect to the vertical line by summing both of the angles. Note that a configuration may be used in which the inertial sensor is provided in the socket 11 or the thigh connection portion 22 to directly measure the thigh angle.

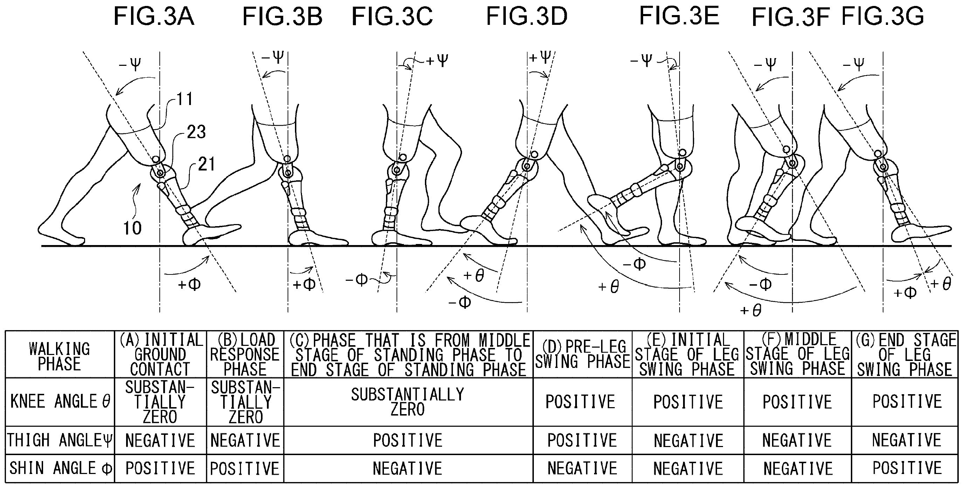

Fig. 3 schematically illustrates transitions of a knee angle θ, a thigh angle Ψ, and a shin angle Φ according to walking phases (A) to (G) of the wearer of the prosthetic leg 10. The walking phase (A) is an initial ground contact (also referred to as an initial contact (IC)), and the prosthetic leg 10 comes into contact with the ground. The walking phase (B) is a load response phase (also referred to as a loading response (LR)), and the weight of the wearer is supported by the prosthetic leg 10 coming into contact with the ground. The walking phase (C) is a phase from a middle stage of a standing phase (also referred to as a mid stance (MSt)) to an end stage of the standing phase (also referred to as a terminal stance (TSt)), and a center of gravity of the wearer moves to the front side of the prosthetic leg 10 in a state in which the prosthetic leg 10 supports the weight. The walking phase (D) is a pre-leg swing phase (also referred to as a pre-swing (PSw)), and the prosthetic leg 10 kicks the ground to transition to the subsequent leg swing phase. The walking phases (E), (F), and (G) are an initial stage of a leg swing phase (also referred to as an initial swing (ISw)), a middle stage of the leg swing phase (also referred to as a mid swing (MSw)), and an end stage of the leg swing phase (also referred to as a terminal swing (TSw)), respectively, and the prosthetic leg 10 that is away from the ground is swung out from the back side to the front side.

The thigh angle Ψ is positive (illustrated as +Ψ) when the socket 11 is inclined forward around the knee shaft 23 with respect to the vertical line, and is negative (illustrated as -Ψ) when the socket 11 is inclined backward around the knee shaft 23 with respect to the vertical line. The shin angle Φ is positive (illustrated as +Φ) when the lower leg portion 21 is inclined forward around the knee shaft 23 with respect to the vertical line, and is negative (illustrated as -Φ) when the lower leg portion 21 is inclined backward around the knee shaft 23 with respect to the vertical line. The knee angle θ is positive (illustrated as +θ) when the lower leg portion 21 is inclined backward around the knee shaft with respect to the axis of the socket 11. The knee angle θ, the thigh angle Ψ, and the shin angle Φ defined as described above always satisfy a relational equation of "θ + Ψ + Φ = 0".

As illustrated in Fig. 3, the knee angle θ is substantially zero / the thigh angle Ψ is negative / the shin angle Φ is positive in the initial ground contact (A), the knee angle θ is substantially zero / the thigh angle Ψ is negative / the shin angle Φ is positive in the load response phase (B), the knee angle θ is substantially zero / the thigh angle Ψ is positive / the shin angle Φ is negative in the phase (C) that is from the middle stage of the standing phase to the end stage of the standing phase, the knee angle θ is positive / the thigh angle Ψ is positive / the shin angle Φ is negative in the pre-leg swing phase (D), the knee angle θ is positive / the thigh angle Ψ is negative / the shin angle Φ is negative in the initial stage of the leg swing phase (E), the knee angle θ is positive / the thigh angle Ψ is negative / the shin angle Φ is negative in the middle stage of the leg swing phase (F), and the knee angle θ is positive / the thigh angle Ψ is negative / the shin angle Φ is positive in the end stage of the leg swing phase (G).

Focusing on the knee angle θ, the knee angle θ is substantially zero in standing phases (A) to (C) in which the prosthetic leg 10 coming into contact with the ground supports the weight of the wearer, the knee angle θ is positive in the pre-leg swing phase (D) between the standing phases (A) to (C) and the leg swing phases (E) to (G), and the knee angle θ monotonically increases or decreases to take the maximum value in the middle stage of the leg swing phase (F) in the leg swing phases (E) to (G) in which the prosthetic leg 10 is away from the ground and swung.

The control of the knee angle θ, that is, the bending control of the knee joint 20 is performed by the control unit 50 via the drive mechanism 40 and the cylinder 30. That is, in the standing phases (A) to (C), hydraulic resistance of the cylinder 30 is increased to restrict the rotation around the knee shaft 23 such that the knee joint 20 is not bent by the weight of the wearer, and thus the knee angle θ is maintained at substantially zero. In the pre-leg swing phase (D), in preparation of the subsequent leg swing phases (E) to (G), the hydraulic resistance of the cylinder 30 is gradually decreased to allow the rotation around the knee shaft 23, and thus the knee angle θ becomes positive such that the knee joint 20 can start to be bent. In the leg swing phases (E) to (G), the hydraulic resistance of the cylinder 30 is maintained to be low and the bending of the knee joint 20 is assisted such that the prosthetic leg 10 away from the ground and swung does not come into contact with the ground.

Returning to Figs. 1 and 2, the description of the prosthetic leg 10 will be continued. As a sensor other than the knee angle sensor 60 and the inertial sensor 75, a load sensor 70 that detects a load applied between the lower leg portion 21 and the foot portion 12 may be provided at the lower end of the lower leg portion 21. Since the walking phase of the wearer of the prosthetic leg 10 can be recognized based on the magnitude and direction of the load detected by the load sensor 70, the load sensor 70 may be used for bending control for the knee joint 20 in addition to the knee angle sensor 60 and the inertial sensor 75. On the other hand, since the bending control for the knee joint 20 can be performed only by the knee angle sensor 60 and the inertial sensor 75, the knee joint 20 can be configured at low cost without using the load sensor 70. Furthermore, a temperature sensor 80 that measures the temperature of oil in the cylinder 30 is attached to the outer wall or the inner wall of the cylinder 30. The measurement information of each of these sensors is used by the control unit 50.

A vibrator 85 is provided in the thigh connection portion 22 or the lower leg portion 21. The vibrator 85 performs notification or calls attention to a user wearing the prosthetic leg 10 by vibration, and is controlled by the control unit 50.

The control unit 50 controls the drive mechanism 40 based on the measurement information obtained by various sensors such as the knee angle sensor 60, the load sensor 70, the inertial sensor 75, and the temperature sensor 80, and controls the resistance to the expansion and contraction operation of the cylinder 30, that is, the rotational resistance of the knee shaft 23 at the time of the bending and stretching motion of the knee joint 20. A battery 55 that supplies power to each unit of the knee joint 20 is connected to the control unit 50. Note that the drive mechanism 40, the control unit 50, and the battery 55 are illustrated outside the knee joint 20 in Fig. 2, but are actually provided inside the lower leg portion 21 as components of the knee joint 20.

The cylinder 30 is a hydraulic cylinder that generates resistance force by using oil as a working fluid to restrict or assist the bending and stretching motion of the knee joint 20. The cylinder 30 is supported by an upper support point 31 provided near the knee shaft 23 rotatably connecting the socket 11 with the lower leg portion 21 and a lower support point 32 connected to a part of the lower leg portion 21, and can expand and contract between both of the support points. In a contraction process in which a cylinder length decreases, the bending motion in which the knee joint 20 rotates in a counterclockwise direction in Fig. 1 around the knee shaft 23 is performed, and in an expansion process in which the cylinder length increases, a stretching motion in which the knee joint 20 rotates in a clockwise direction in Fig. 1 around the knee shaft 23 is performed. Here, the cylinder length refers to a length between the upper support point 31 and the lower support point 32 of the cylinder 30.

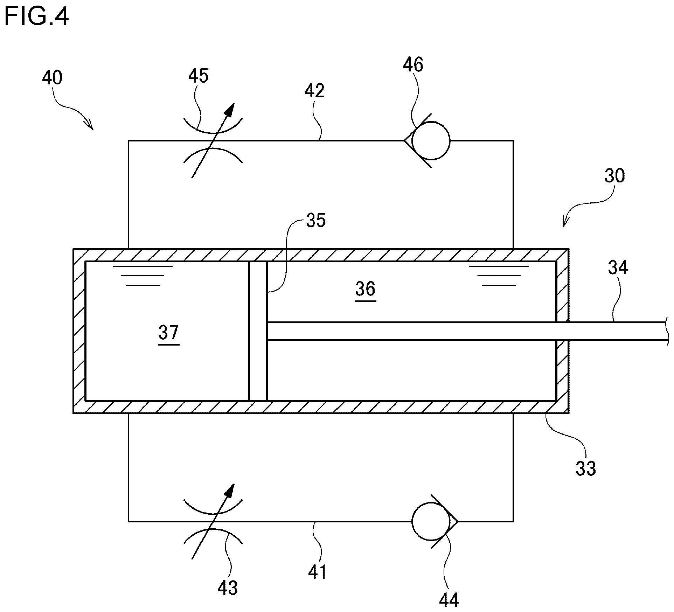

Next, the cylinder 30 and the drive mechanism 40 will be described with reference to Fig. 4. The cylinder 30 includes a cylinder tube 33, the piston rod 34 that is inserted from one end side (right end side in Fig. 4) of the cylinder tube 33 and is movable along a longitudinal direction (right and left direction in Fig. 4) of the cylinder tube 33, and a piston 35 that is fixed to the piston rod 34 in the cylinder tube 33 and is slidable in the longitudinal direction along an inner wall of the cylinder tube 33. The inside of the cylinder tube 33 is divided, by the piston 35, into a first cavity 36 on one end side (right end side in Fig. 4) and a second cavity 37 on the other end side (left end side in Fig. 4). The first cavity 36 and the second cavity 37 are filled with the oil that is the working fluid.

The drive mechanism 40 is a hydraulic drive mechanism that drives the cylinder 30 to expand and contract by hydraulic pressure. The drive mechanism 40 includes a stretching-side hydraulic circuit 41 and a bending-side hydraulic circuit 42, which are connected to the cylinder 30. The stretching-side hydraulic circuit 41 and the bending-side hydraulic circuit 42 communicate with the first cavity 36 on one end side and with the second cavity 37 on the other end side, respectively. The stretching-side hydraulic circuit 41 includes a stretching-side valve 43 as a valve capable of opening and closing a flow passage of oil that generates the rotational resistance of the knee shaft 23, and a stretching-side check valve 44. When the stretching-side valve 43 is opened, the oil can flow through the stretching-side hydraulic circuit 41, but due to an action of the stretching-side check valve 44, the oil flows only in a direction from the first cavity 36 toward the second cavity 37 and does not flow in the opposite direction.

The bending-side hydraulic circuit 42 includes a bending-side valve 45 as a valve capable of opening and closing the flow passage of oil that generates the rotational resistance of the knee shaft 23, and a bending-side check valve 46. When the bending-side valve 45 is opened, the oil can flow through the bending-side hydraulic circuit 42, but due to an action of the bending-side check valve 46, the oil flows only in a direction from the second cavity 37 toward the first cavity 36 and does not flow in the opposite direction. The opening degrees of the stretching-side valve 43 and the bending-side valve 45 are individually controlled by the control unit 50. Any value of the opening degree of each valve can be obtained among values obtained between a fully open state (maximum opening degree) and a fully closed state (minimum opening degree). When each valve is fully closed, the flow of oil is blocked, and the hydraulic resistance is maximized. Furthermore, as the opening degree of each valve increases toward the full opening, the cross-sectional area through which the oil can flow in each valve increases. Therefore, the flow rate of the oil increases and the hydraulic resistance decreases.

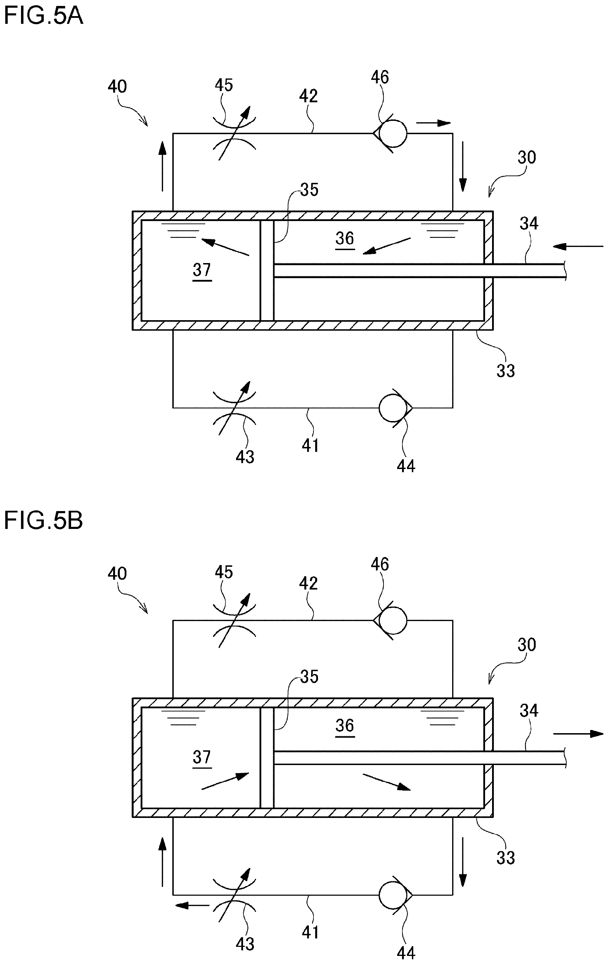

Fig. 5A is a diagram illustrating the flow of oil at the time of the bending motion of the knee joint 20. The bending is a retracting process in which the cylinder length decreases, the piston rod 34 is retracted to the left side in Fig. 5A, and the piston 35 moves to the retracting side. Since the oil pushed out from the second cavity 37 by moving the piston 35 cannot flow through the stretching-side hydraulic circuit 41 having the stretching-side check valve 44, the oil flows through the bending-side hydraulic circuit 42 and flows into the first cavity 36. At this time, when the opening degree of the bending-side valve 45 is lowered, the oil can be made difficult to flow through the bending-side hydraulic circuit 42. Therefore, the bending motion of the knee joint 20 can be restricted. As described above, the control unit 50, the drive mechanism 40, and the cylinder 30 constitute a rotational resistor of the present invention that applies resistance caused by hydraulic pressure to the rotation of the knee shaft 23 at the time of the bending motion of the knee joint 20.

Fig. 5B illustrates the flow of oil at the time of the stretching motion of the knee joint 20. The stretching is an extension process in which the cylinder length increases, the piston rod 34 is extended to the right side in Fig. 5B, and the piston 35 moves to the extrusion side. Since the oil pushed out from the first cavity 36 by moving the piston 35 cannot flow through the bending-side hydraulic circuit 42 having the bending-side check valve 46, the oil flows through the stretching-side hydraulic circuit 41 and flows into the second cavity 37. At this time, when the opening degree of the stretching-side valve 43 is lowered, the oil can be made difficult to flow through the stretching-side hydraulic circuit 41. Therefore, the stretching motion of the knee joint 20 can be restricted. As described above, the control unit 50, the drive mechanism 40, and the cylinder 30 constitute a rotational resistor of the present invention that applies resistance caused by hydraulic pressure to the rotation of the knee shaft 23 at the time of the stretching motion of the knee joint 20.

Returning to Fig. 2, additional descriptions for various sensors provided in the prosthetic leg 10 will be made.

The knee angle sensor 60 measures an extending and retreating position of the piston rod 34. For example, the position of the magnet attached to the piston rod 34 is measured by a magnetic sensor provided in the cylinder tube 33. Since the extending and retreating position of the piston rod 34 and the knee angle of the knee joint 20 or the rotation angle of the knee shaft 23 are in one-to-one correspondence, the knee angle sensor 60 can convert the detected extending and retreating position of the piston rod 34 into the knee angle of the knee joint 20. Note that calculation for converting the extending and retreating position of the piston rod 34 into the knee angle of the knee joint 20 may be performed by the control unit 50. In this case, the knee angle sensor 60 measures the extending and retreating position of the piston rod 34 and provides the measurement result to the control unit 50.

The inertial sensor 75 detects the orientation and motion of the lower leg portion 21 based on the measured speed and/or acceleration of each axis. For example, the walking phase of the wearer of the prosthetic leg 10 can be recognized from the change in the speed and/or acceleration measured by the inertial sensor 75, and the position of the lower leg portion 21 during walking can be tracked by integrating the measurement values of the inertial sensor 75. Therefore, a stride which is a displacement amount per step and the walking speed per step can be obtained. Note that calculation of integrating the measurement values of the inertial sensor 75 to obtain the position is performed by the control unit 50.

The load sensor 70 includes, for example, a strain sensor, and is provided at the ankle portion between the lower leg portion 21 and the foot portion 12. Since the load applied to the ankle portion causes strain in the object constituting the strain sensor, the load can be measured by detecting the strain. When such a load sensor is provided in the thigh connection portion 22, a load applied to the knee joint can also be measured. Note that calculation of converting the strain detected by the strain sensor into a load may be performed by the control unit 50. In this case, the load sensor 70 provides the detected strain to the control unit 50. Furthermore, since the control unit 50 can recognize the walking phase of the wearer of the prosthetic leg 10 from the change in the magnitude and direction of the load measured by the load sensor 70, the stride and the walking speed can be obtained by the calculation.

The temperature sensor 80 measures the temperature of the oil that is the working fluid of the cylinder 30. Since the hydraulic resistance changes according to the temperature change depending on the physical properties of the oil, the control unit 50 controls the drive mechanism 40 according to the temperature measured by the temperature sensor 80 to realize a desired hydraulic resistance. Specifically, a control data set for the drive mechanism 40 for obtaining each value of the hydraulic resistance is created for each temperature and stored in the control unit 50. The control unit 50 selects a control data set corresponding to the temperature measured by the temperature sensor 80 and uses the control data set for control.

In the electronically controlled passive type of knee joint 20 as described above, it is possible to configure a knee angle restrictor that restricts the relative rotation of the thigh connection portion 22 and the lower leg portion 21 such that the knee angle θ formed by the thigh connection portion 22 with respect to the lower leg portion 21 does not exceed a predetermined lock angle.

Specifically, in a case where the knee angle θ measured by the knee angle sensor 60 increases to a predetermined lock angle θ_{L} at the time of the bending motion of the knee joint 20 illustrated in Fig. 5A, the control unit 50 causes the bending-side valve 45 into the fully closed state (minimum opening degree). According to this, since the flow of oil in the bending-side hydraulic circuit 42 is blocked, the rotation of the knee shaft 23 beyond the lock angle θ_{L} is restricted. In this locked state, it is possible to prevent the knee bending beyond the lock angle θ_{L}, which is not intended by the wearer of the knee joint 20. Note that although the magnitude of the lock angle θ_{L} can be arbitrarily set, it is preferable to set the lock angle θ_{L} typically in a range of 10 degrees to 40 degrees so as not to hinder normal walking as illustrated in Fig. 3.

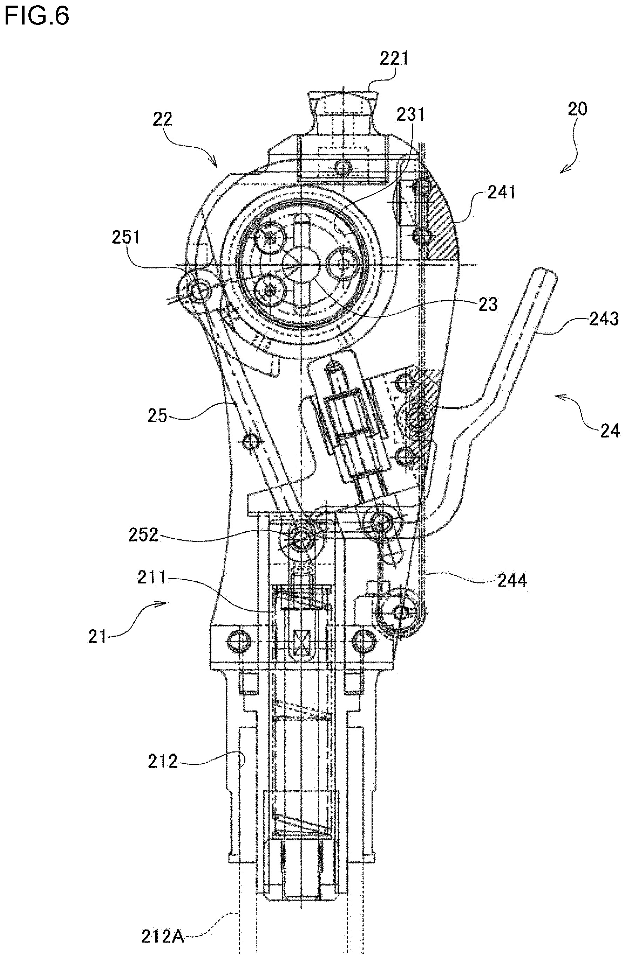

On the other hand, when the wearer needs to sit by bending the knee at an angle greater than the lock angle θ _{L}, the control unit 50 that detects the sitting-motion based on the operation of the wearer of the knee joint 20 and the data measured by the inertial sensor 75 maintains the open state of the bending-side valve 45 even in a case where the knee angle θ increases to the lock angle θ_{L}. According to this, since the oil can flow through the bending-side hydraulic circuit 42, the rotation of the knee shaft 23 beyond the lock angle θ_{L} is allowed, and the sitting motion in which the knee is largely bent can be performed. Such switching between the locked state and the unlocked state is electrically performed by electronic control of the control unit 50 as an electrical switcher. Note that the lock mechanism of the knee angle θ may be mechanically realized by providing a mechanical knee angle restrictor 24 to be described later in Figs. 6 to 13 to the electronically controlled knee joint 20 of Figs. 1 and 2.

In contrast to JP H09-75382 A in which the knee joint 20 can be freely bent in a state in which the knee angle θ is equal to or less than the lock angle θ_{L}, in the present embodiment, even in a state in which the knee angle θ is equal to or less than the lock angle θ_{L}, resistance is applied to the rotation of the knee shaft 23 at the time of the bending motion by the bending-side hydraulic circuit 42 constituting the rotational resistor. The resistance applied by the rotational resistor can prevent a sudden knee bending having the lock angle θ_{L} from occurring from a state in which the knee angle θ, at which the socket 11 and the lower leg portion 21 are on a straight line, is zero degrees, for example. Therefore, according to the present embodiment, it is possible to reduce the falling down risk and anxiety of the wearer of the knee joint 20, which are caused by the sudden knee bending.

In the above configuration of the rotational resistor, the bending-side hydraulic circuit 42 can apply the resistance caused by the hydraulic pressure to the rotation of the knee shaft 23 in the entire angle range in which the knee angle θ is equal to or less than the lock angle θ_{L}. The magnitude of the resistance applied to the rotation of the knee shaft 23 by the bending-side hydraulic circuit 42 is preferably changed according to the walking phase and the knee angle θ in Fig. 3, but may be constant regardless of the walking phase and the knee angle θ. In this case, the opening degree of the bending-side valve 45 in the bending-side hydraulic circuit 42 is kept constant.

The bending-side hydraulic circuit 42 may change the magnitude of the resistance applied to the rotation of the knee shaft 23 according to the load position of the lower leg portion 21 that can be detected by the inertial sensor 75 and the load sensor 70. For example, in Fig. 3, in the initial ground contact (A) in which the load position is near a heel, the resistance applied to the rotation of the knee shaft 23 is increased, and the knee angle θ is maintained at substantially zero from the subsequent load response phase (B) to the phase (C) that is from the middle stage of the standing phase to the end stage of the standing phase. Furthermore, in the pre-leg swing phase (D) in which the load position is near a tiptoe, the resistance applied to the rotation of the knee shaft 23 is reduced such that the knee angle θ can be greatly changed from the subsequent initial stage of the leg swing phase (E) to the middle stage of the leg swing phase (F). In this case, the bending-side hydraulic circuit 42 decreases the resistance applied to the rotation of the knee shaft 23 according to an increase in the knee angle θ from the phase (C) that is from the middle stage of the standing phase to the end stage of the standing phase in which the knee angle θ is substantially zero to the pre-leg swing phase (D) in which the knee angle θ is positive. Since the resistance is minimized immediately before the lock angle θ_{L}, the knee joint 20 can smoothly transition to the locked state while suppressing the knee bending. On the other hand, the bending-side hydraulic circuit 42 may increase the resistance applied to the rotation of the knee shaft 23 according to the increase in the knee angle θ. Since the resistance is maximized immediately before the lock angle θ_{L}, the impact due to the sudden knee bending can be effectively decreased. As described above, by changing the magnitude of the resistance applied to the rotation of the knee shaft 23, the bending-side hydraulic circuit 42 can prevent the sudden knee bending while securing the ease of walking according to the walking phase and the ease of locking the knee joint 20.

The bending-side hydraulic circuit 42 may change the magnitude of the resistance applied to the rotation of the knee shaft 23 according to a knee angular velocity obtained by the control unit 50 time-differentiating the knee angle θ measured by the knee angle sensor 60 (angular velocity detector). For example, in Fig. 3, in the initial ground contact (A) to the phase (C) that is from the middle stage of the standing phase to the end stage of the standing phase in which the knee angular velocity is substantially zero, the resistance applied to the rotation of the knee shaft 23 is increased, and thus the bending of the knee is restricted Furthermore, in the pre-leg swing phase (D) to the middle stage of the leg swing phase (F) in which the knee angular velocity is positive, the resistance applied to the rotation of the knee shaft 23 is decreased, and thus the bending of the knee is assisted. In this case, the bending-side hydraulic circuit 42 decreases the resistance applied to the rotation of the knee shaft 23 according to an increase in the knee angular velocity from the phase (C) that is from the middle stage of the standing phase to the end stage of the standing phase in which the knee angular velocity is substantially zero to the pre-leg swing phase (D) in which the knee angular velocity is positive. On the other hand, the bending-side hydraulic circuit 42 may increase the resistance applied to the rotation of the knee shaft 23 according to the increase in the knee angular velocity.

As described above, the electronically controlled passive type of knee joint 20 has been described. Next, the mechanical passive type of knee joint 20 will be described.

Fig. 6 illustrates a first configuration example of the mechanical passive type of knee joint 20. The knee joint 20 includes the thigh connection portion 22 to which the socket 11 (not illustrated) is connected, the lower leg portion 21 connected to the thigh connection portion 22 and rotatably provided around a knee shaft 23, and the knee angle restrictor 24 that restricts the relative rotation of the thigh connection portion 22 and the lower leg portion 21. The knee shaft 23 is located substantially at the center of the thigh connection portion 22, and is provided with an accommodation space 231 having a circular cross section illustrated in Fig. 6, the accommodation space 231 being capable of accommodating a bearing that rotatably supports the knee shaft 23, and a rotational resistor that applies the resistance to rotation of the knee shaft 23 or the rotational resistance module.

The thigh connection portion 22 and the lower leg portion 21 are connected by a long link 25. Specifically, an upper end portion of the link 25 is fixed to the thigh connection portion 22 at a thigh connection portion 251 provided on the outer periphery of the accommodation space 231, and a lower end portion of the link 25 is fixed to the lower leg portion 21 at a lower leg connection portion 252 located substantially at the center of the lower leg portion 21. Note that, for convenience of the illustration, only one link 25 is illustrated, but a plurality of the links 25 may be arranged side by side along a direction perpendicular to the paper surface. In this configuration, the thigh connection portion 22 is rotatable around the knee shaft 23 in a state of being connected to the lower leg portion 21 by the link 25. In Fig. 6, the operation in which the thigh connection portion 22 rotates in the counterclockwise direction around the knee shaft 23 corresponds to the bending motion of the knee joint 20, and the operation in which the thigh connection portion 22 rotates in the clockwise direction around the knee shaft 23 corresponds to the stretching motion of the knee joint 20.

The lower leg portion 21 is provided with a stretching assist spring 211 that biases the lower leg connection portion 252 of the link 25 upward along the axis of the lower leg portion 21. The stretching assist spring 211 biases the thigh connection portion 22 in the clockwise direction, that is, in the stretching direction of the knee joint 20 via the link 25. According to this, when the knee joint 20 is slightly bent, the knee joint 20 can be naturally returned to the stretched state by biasing force of the stretching assist spring 211. Furthermore, also when the knee joint 20 returns from a state in which the wearer sits by bending the knee joint 20 greatly in the unlocked state, the stretching assist spring 211 acts to assist the stretching of the knee joint 20.

An attachment pipe 212A (broken line) having the foot portion 12 provided at the lower end is inserted into a foot portion attachment hole 212 provided on a lower side of the stretching assist spring 211 of the lower leg portion 21. Furthermore, the socket 11 as the thigh portion is attached to a thigh attachment portion 221 provided at the upper end of the thigh connection portion 22. In this manner, by attaching the foot portion 12 and the socket 11 to the knee joint 20, the prosthetic leg 10 illustrated in Fig. 1 is configured.

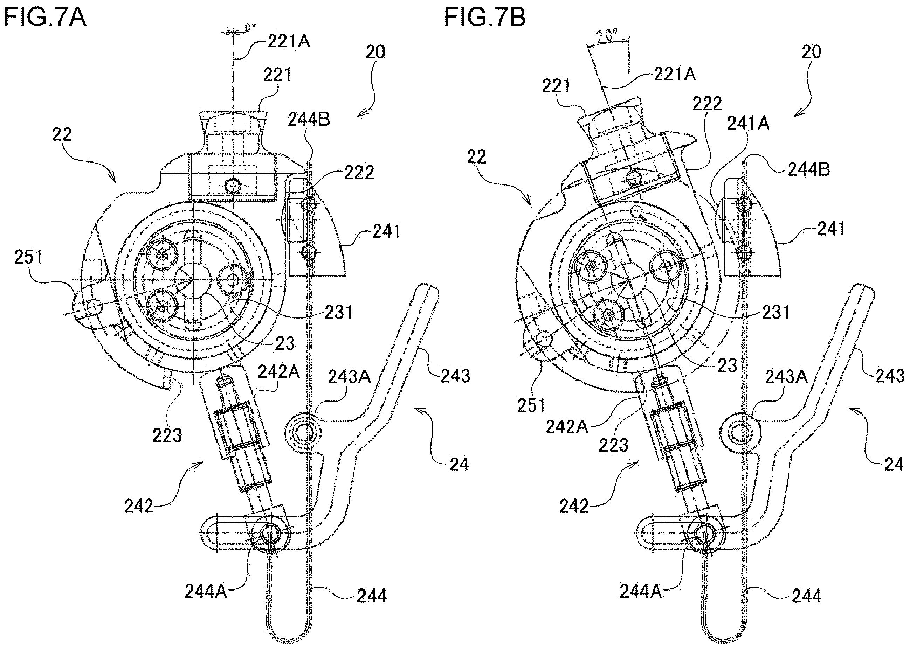

Figs. 7A and 7B illustrate the thigh connection portion 22 and the knee angle restrictor 24 for illustrating the rotational operation around the knee shaft 23, that is, the bending and stretching motion of the knee joint 20. Fig. 7A illustrates a state in which the knee angle θ, at which an axis 221A of the thigh attachment portion 221 of the thigh connection portion 22 and an axis of the lower leg portion 21 (not illustrated) are on a straight line (on a vertical line in Fig. 7A), is zero degrees. Fig. 7B illustrates a state in which a direction of the axis of the lower leg portion 21 is maintained (vertical direction in Fig. 7B), and the knee angle θ obtained by bending the axis 221A of the thigh attachment portion 221 of the thigh connection portion 22 by 20 degrees in the counterclockwise direction is 20 degrees.

The knee angle restrictor 24 is fixed to the lower leg portion 21 (not illustrated), and restricts the relative rotation of the thigh connection portion 22 and the lower leg portion 21 such that the knee angle θ formed by the thigh connection portion 22 with respect to the lower leg portion 21 does not exceed a predetermined lock angle θ_{L}. In the illustrated example, the lock angle θ_{L} is 20 degrees. Therefore, in the locked state in which the knee angle restrictor 24 restricts the knee angle θ to the lock angle θ_{L} or less, a value obtained between zero degrees in Fig. 7A and 20 degrees in Fig. 7B can be acquired as the knee angle θ, but a value outside this range cannot be acquired.

In Fig. 7A in which the knee angle θ is the minimum (zero degrees), a stretching-side contact portion 222 provided on the stretching side (clockwise side in Fig. 7A) on the outer periphery of the thigh connection portion 22 is stopped by coming into contact with a stretching-side stopping member 241 of the knee angle restrictor 24. The stretching-side contact portion 222 and the stretching-side stopping member 241 form a shape to be engaged with each other, and an elastic member 241A formed of an elastic material on a contact surface of the stretching-side stopping member 241 alleviates the impact caused by the contact between the stretching-side contact portion 222 and the stretching-side stopping member 241. In the state of Fig. 7A in which the stretching-side contact portion 222 and the stretching-side stopping member 241 are engaged, the thigh connection portion 22 is stopped by the stretching-side stopping member 241 and cannot rotate in the clockwise direction (stretching direction) any more. Therefore, the knee angle θ is restricted not to be smaller than zero degrees in Fig. 7A.

In Fig. 7B in which the knee angle θ is the maximum (lock angle θ_{L} = 20 degrees), a bending-side contact portion 223 provided on the bending side (counterclockwise side in Fig. 7B) on the outer periphery of the thigh connection portion 22 is stopped by coming into contact with a bending-side stopping member 242 of the knee angle restrictor 24. The bending-side contact portion 223 as a rotating member that rotates integrally with the thigh connection portion 22 is formed as an end surface of the thigh connection portion 22 that advances in the counterclockwise direction according to the bending motion of the knee joint 20, and comes into surface contact with the bending-side stopping member 242 on the path when the knee angle θ is the lock angle θ_{L} = 20 degrees. A bending-side stopping portion 242A of the bending-side stopping member 242 is formed of a metal material or an elastic material. When the bending-side stopping portion 242A is formed of the elastic material, the impact at the time of the contact with the bending-side contact portion 223 can be alleviated. In the state of Fig. 7B in which the bending-side contact portion 223 and the bending-side stopping portion 242A come into surface contact with each other, the thigh connection portion 22 is stopped by the bending-side stopping member 242 and cannot rotate in the counterclockwise direction (bending direction) any more. Therefore, the knee angle θ is restricted not to be greater than 20 degrees in Fig. 7B.

The knee angle restrictor 24 includes a mechanical switcher that mechanically performs switching between a locked state in which the knee angle restrictor 24 restricts the rotation of the knee shaft 23 and an unlocked state in which the knee angle restrictor 24 does not restrict the rotation of the knee shaft 23. Although two types of mechanical switchers are illustrated together in Figs. 7A and 7B, it is sufficient to provide one of the mechanical switchers in practice.

A lever 243 as a first mechanical switcher can be rotated centered on a fulcrum 243A fixed to the lower leg portion 21 by a manual operation of the wearer of the knee joint 20. By the rotation operation of the lever 243, the bending-side stopping member 242 moves away from the knee shaft 23 in a radial direction, and deviates from the course of the bending-side contact portion 223 of the thigh connection portion 22. As a result, the thigh connection portion 22 is switched to the unlocked state of being rotated in the counterclockwise direction (bending direction) beyond the lock angle θ_{L}.

One end 244A of a wire 244 as a second mechanical switcher is connected to the bending-side stopping portion 242A, and the wearer of the knee joint 20 can pull the other end 244B. By the pulling operation of the other end 244B of the wire 244, the bending-side stopping portion 242A connected to one end 244A is pulled, moves away from the knee shaft 23 in a radial direction, and deviates from the course of the bending-side contact portion 223 of the thigh connection portion 22. As a result, the thigh connection portion 22 is switched to the unlocked state of being rotated in the counterclockwise direction (bending direction) beyond the lock angle θ_{L}.

In contrast to JP H09-75382 A in which the knee joint 20 can be freely bent in a state in which the knee angle θ is equal to or less than the lock angle θ_{L}, in the present configuration example, the resistance is applied to the rotation of the knee shaft 23 at the time of the bending motion by accommodating the rotational resistor or the rotational resistance module in the accommodation space 231 even in a state in which the knee angle θ is equal to or less than the lock angle θ_{L}. Due to the resistance applied by the rotational resistor or the rotational resistance module, for example, it is possible to prevent the sudden knee bending having the lock angle θ_{L} (Fig. 7B) from occurring from the state in which the knee angle θ, at which the axis 221A of the thigh attachment portion 221 of the thigh connection portion 22 and the axis of the lower leg portion 21 are on a straight line, is zero degrees (Fig. 7A). Therefore, according to the present configuration example, it is possible to reduce the falling down risk and anxiety of the wearer of the knee joint 20, which are caused by the sudden knee bending.

The type of the rotational resistor or rotational resistance module accommodated in the accommodation space 231 is not limited as long as the resistance can be applied to the rotation of the knee shaft 23. For example, the resistance may be applied to the rotation of the knee shaft 23 by the hydraulic pressure, the resistance may be applied to the rotation of the knee shaft 23 by viscosity of a viscous fluid made of grease or polymer, the resistance may be applied to the rotation of the knee shaft 23 by mechanical friction, a speed reducer that decelerates the rotation of the knee shaft 23 by using a gear may be used, or a motor that gives rotational power to the knee shaft 23 may be used. By configuring these rotational resistors as the rotational resistance module detachable from the accommodation space 231, it is possible to select an optimum type of rotational resistance module for the wearer for each knee joint 20. Furthermore, since the restriction for a medium included in the rotational resistor varies depending on the country and region, the rotational resistor is configured as a module that can be retrofitted to the knee joint 20. Therefore, it is possible to reduce the time and effort to customize the knee joint 20 for each country and region.

As described above, the rotational resistor or the rotational resistance module can apply the resistance to the rotation of the knee shaft 23 in the entire angle range in which the knee angle θ is equal to or less than the lock angle θ_{L}. The magnitude of the resistance applied to the rotation of the knee shaft 23 by the rotational resistor or the rotational resistance module is preferably changed according to the walking phase and the knee angle θ in Fig. 3, but may be constant regardless of the walking phase and the knee angle θ.

Unlike the electronically controlled knee joint 20 illustrated in Figs. 1 to 5B, the mechanical knee joint 20 illustrated in Figs. 6 to 7B is basically not provided with sensors that electrically generate measurement data, such as the knee angle sensor 60, the inertial sensor 75, and the load sensor 70. On the other hand, as in JP 2004-167106 A, a load position detection mechanism that mechanically detects a load position in the lower leg portion 21 is known, and can also be used in the mechanical knee joint 20 of the present embodiment. The example of changing the magnitude of the resistance applied to the rotation of the knee shaft 23 according to the detected load position has been described above with respect to the electronically controlled knee joint 20 in Figs. 1 to 5B, and thus overlapped description will be omitted.

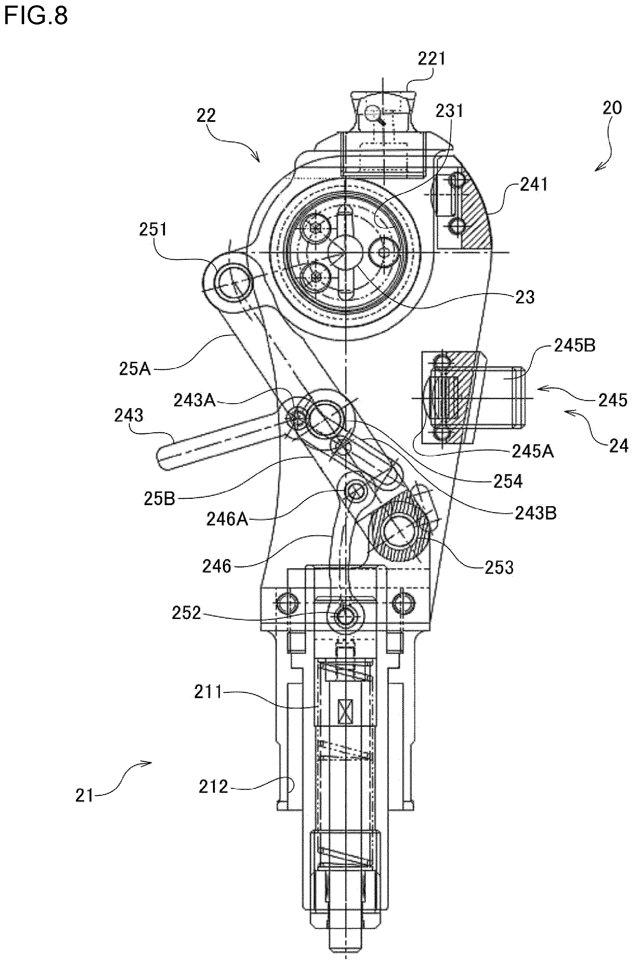

Fig. 8 illustrates a second configuration example of the mechanical passive type of knee joint 20. The same constituent elements as those in the above-described configuration example are denoted by the same reference numerals, and the description thereof will be appropriately omitted. The thigh connection portion 22 and the lower leg portion 21 are connected by a link mechanism including a first link 25A and a second link 25B. One end (upper end) of the first link 25A on the thigh connection portion 22 side is fixed to the thigh connection portion 22 at the thigh connection portion 251, and one end (lower end) of the second link 25B on the lower leg portion 21 side is fixed to the lower leg portion 21 at a lower leg connection portion 253 located in front of the center of the lower leg portion 21 (right side in Fig. 8). The other ends of the first link 25A and the second link 25B are rotatably connected to each other at a link connection portion 254. In this configuration, the thigh connection portion 22 is rotatable around the knee shaft 23 in a state of being connected to the lower leg portion 21 by the first link 25A and the second link 25B. Furthermore, each of end portions of a long support member 246 is connected to the lower leg connection portion 252 of the lower leg portion 21 and a link connection portion 246A of the second link 25B. The stretching assist spring 211 biasing the lower leg connection portion 252 upward biases the thigh connection portion 22 in the stretching direction of the knee joint 20 via the support member 246, the second link 25B, and the first link 25A which are connected to each other.

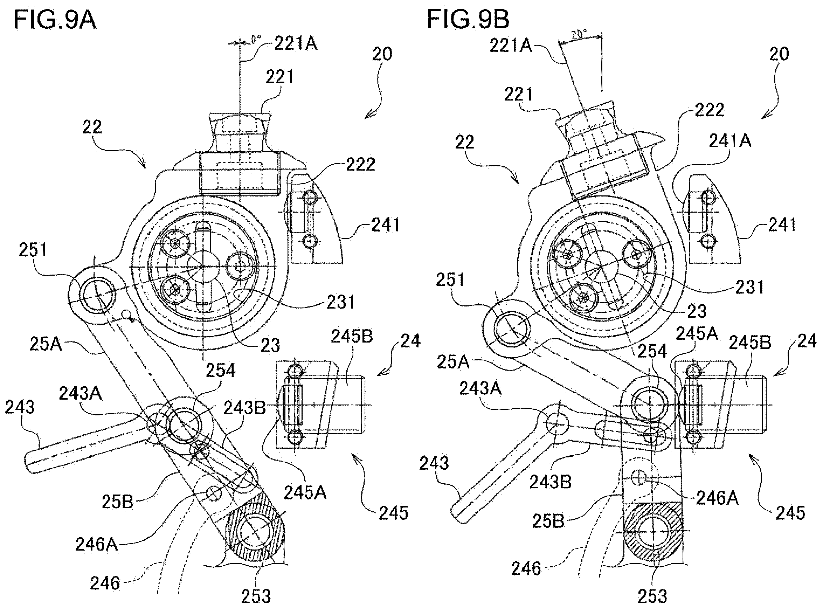

Figs. 9A and 9B illustrate the thigh connection portion 22 and the knee angle restrictor 24 for illustrating the rotational operation around the knee shaft 23, that is, the bending and stretching motion of the knee joint 20. Fig. 9A illustrates a state in which the knee angle θ, at which an axis 221A of the thigh attachment portion 221 of the thigh connection portion 22 and an axis of the lower leg portion 21 (not illustrated) are on a straight line (on a vertical line in Fig. 9A), is zero degrees. Fig. 9B illustrates a state in which a direction of the axis of the lower leg portion 21 is maintained (vertical direction in Fig. 9B), and the knee angle θ obtained by bending the axis 221A of the thigh attachment portion 221 of the thigh connection portion 22 by 20 degrees in the counterclockwise direction is 20 degrees.

The knee angle restrictor 24 is fixed to the lower leg portion 21 (not illustrated), and restricts the relative rotation of the thigh connection portion 22 and the lower leg portion 21 such that the knee angle θ formed by the thigh connection portion 22 with respect to the lower leg portion 21 does not exceed a predetermined lock angle θ_{L}. In the illustrated example, the lock angle θ_{L} is 20 degrees. Therefore, in the locked state in which the knee angle restrictor 24 restricts the knee angle θ to the lock angle θ_{L} or less, a value obtained between zero degrees in Fig. 9A and 20 degrees in Fig. 9B can be acquired as the knee angle θ, but a value outside this range cannot be acquired.

In Fig. 9B in which the knee angle θ is the maximum (lock angle θ_{L} = 20 degrees), the link connection portion 254 having the link mechanism and having been bent according to the rotation of the knee shaft 23 is stopped by coming into contact with a bending-side stopping member 245 of the knee angle restrictor 24. When the thigh connection portion 22 rotates in the counterclockwise direction (bending direction) around the knee shaft 23 according to the bending of the knee joint 20 in Figs. 9A and 9B, the link connection portion 254 moves forward toward the bending-side stopping member 245 by being guided by a guide portion 243B in front of the fulcrum 243A of the lever 243 (right side in Fig. 9A).

In the state of Fig. 9B in which the link connection portion 254 and the bending-side stopping member 245 come into surface contact with each other, the thigh connection portion 22 is stopped by the bending-side stopping member 245 and cannot rotate in the counterclockwise direction (bending direction) any more. Therefore, the knee angle θ is restricted not to be greater than 20 degrees in Fig. 9B.

The bending-side stopping member 245 with which the link connection portion 254 comes into contact at the lock angle includes an elastic member 245A that is provided on the contact surface and formed of an elastic material to alleviate the impact caused by the contact, and a position change portion 245B for changing a position of the elastic member 245A. The position change portion 245B is configured by a screw in which the elastic member 245A is embedded in a distal end surface (left end surface in Fig. 9A and 9B), and the position of the elastic member 245A in the right and left direction in Figs. 9A and 9B can be adjusted by turning the screw. As illustrated in Fig. 9B, since the position of the elastic member 245A determines the lock angle θ_{L}, the position change portion 245B can change the lock angle θ_{L} according to the wearer of the knee joint 20. Note that in the above-described example, the link connection portion 254 of the first link 25A and the second link 25B comes into contact with the bending-side stopping member 245 when the knee angle θ is the lock angle θ_{L}, but is not limited to the link connection portion 254, and any portion of the first link 25A or the second link 25B may come into contact with the bending-side stopping member 245.

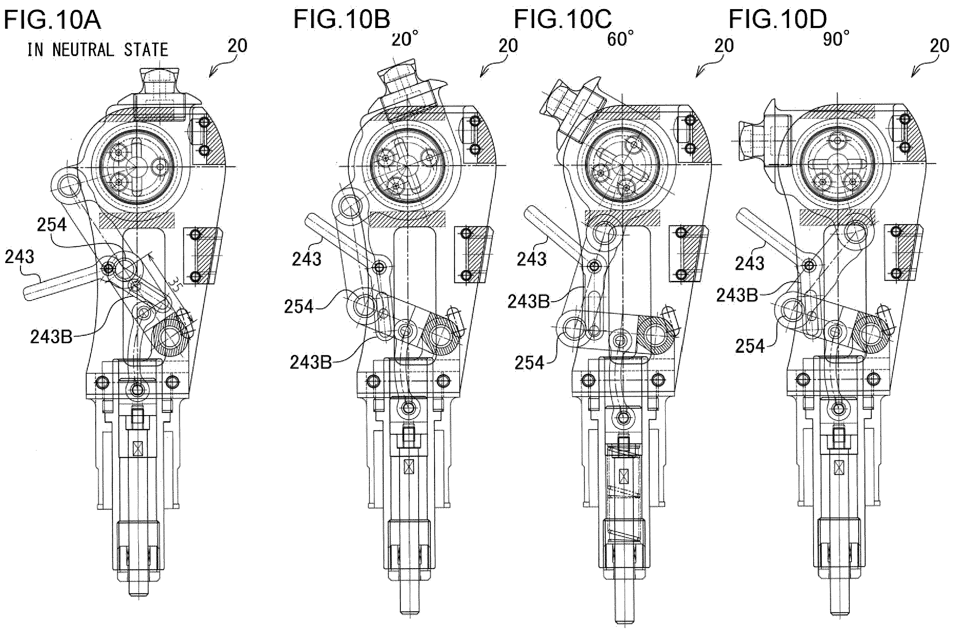

Similarly to the first configuration example in Figs. 6 to 7B, the lever 243 of the knee angle restrictor 24 configures a mechanical switcher that mechanically performs switching between a locked state in which the knee angle restrictor 24 restricts the rotation of the knee shaft 23 and an unlocked state in which the knee angle restrictor 24 does not restrict the rotation of the knee shaft 23. Figs. 10A to 10D illustrate a rotational operation of the knee shaft 23 in the unlocked state. As illustrated in Figs. 10B to 10D in which the knee angle θ is equal to or greater than the lock angle θ_{L} = 20 degrees, the link connection portion 254 is moved to the inside of an inverted "V" shape of the lever 243 by the manual operation of the lever 243 of the wearer of the knee joint 20. In this state, since the guide surface of the guide portion 243B on the side opposite to Figs. 8 to 9B guides the link connection portion 254, the link mechanism including the first link 25A and the second link 25B is bent in the opposite direction to that in Fig. 9B. That is, in Fig. 9B, the link mechanism is bent in the inverted "V" shape, but in Figs. 10B to 10D, the link mechanism is bent in a "<" shape or a "V" shape. Unlike the locked state in Fig. 9B, in the unlocked state in Fig. 10, the link connection portion 254 does not come into contact with the bending-side stopping member 245 (not illustrated in Figs. 10A to 10D), and thus the bending motion of the knee joint 20 beyond the lock angle θ _{L} can be performed.

In contrast to JP H09-75382 A in which the knee joint 20 can be freely bent in a state in which the knee angle θ is equal to or less than the lock angle θ_{L}, in the present configuration example, the resistance is applied to the rotation of the knee shaft 23 at the time of the bending motion by accommodating the rotational resistor or the rotational resistance module in the accommodation space 231 even in a state in which the knee angle θ is equal to or less than the lock angle θ_{L}. Due to the resistance applied by the rotational resistor or the rotational resistance module, for example, it is possible to prevent the sudden knee bending having the lock angle θ_{L} (Fig. 9B) from occurring from the state in which the knee angle θ, at which the axis 221A of the thigh attachment portion 221 of the thigh connection portion 22 and the axis of the lower leg portion 21 are on a straight line, is zero degrees (Fig. 9A). Therefore, according to the present configuration example, it is possible to reduce the falling down risk and anxiety of the wearer of the knee joint 20, which are caused by the sudden knee bending.

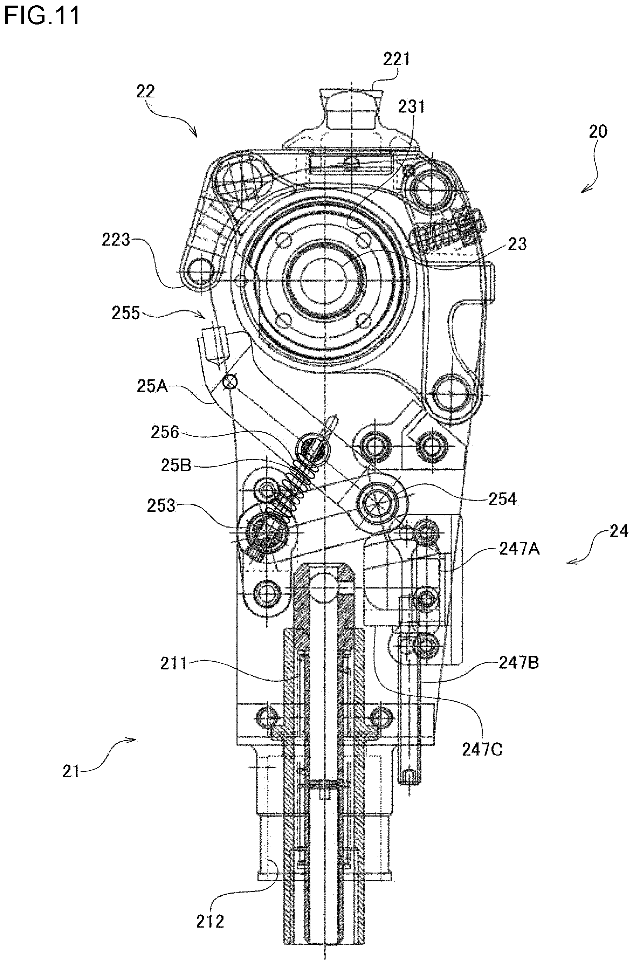

Fig. 11 illustrates a third configuration example of the mechanical passive type of knee joint 20. The same constituent elements as those in the above-described configuration example are denoted by the same reference numerals, and the description thereof will be appropriately omitted. The thigh connection portion 22 and the lower leg portion 21 are connected by a link mechanism including a first link 25A and a second link 25B. An upper end portion 255 on one end side (upper end side) of the first link 25A is not fixed to the thigh connection portion 22, and is smoothly movable with respect to the thigh connection portion 22 while being in contact with the outer periphery of the knee shaft 23 having a circular cross section in Fig. 11. One end (left end) of the second link 25B is fixed to the lower leg portion 21 in the lower leg connection portion 253 located behind the center of the lower leg portion 21 (left side in Fig. 11). The other ends of the first link 25A and the second link 25B are rotatably connected to each other at a link connection portion 254. In this configuration, the thigh connection portion 22 is rotatable around the knee shaft 23 in a state of being connected to the lower leg portion 21 by the first link 25A and the second link 25B.

The link mechanism is provided with a spring 256 having one end (upper right end) connected to substantially the center of the first link 25A and the other end (lower left end) connected to the lower leg connection portion 253 of the second link 25B. Since the first link 25A is biased upward from the spring 256 provided immediately below the first link 25A, the upper end portion 255 of the first link 25A is in a state of being constantly pressed against the outer periphery of the knee shaft 23. In this manner, the upper end portion 255 of the first link 25A biases the thigh connection portion 22 including the knee shaft 23 in the clockwise direction, that is, the stretching direction of the knee joint 20 (direction in which the knee angle θ decreases).

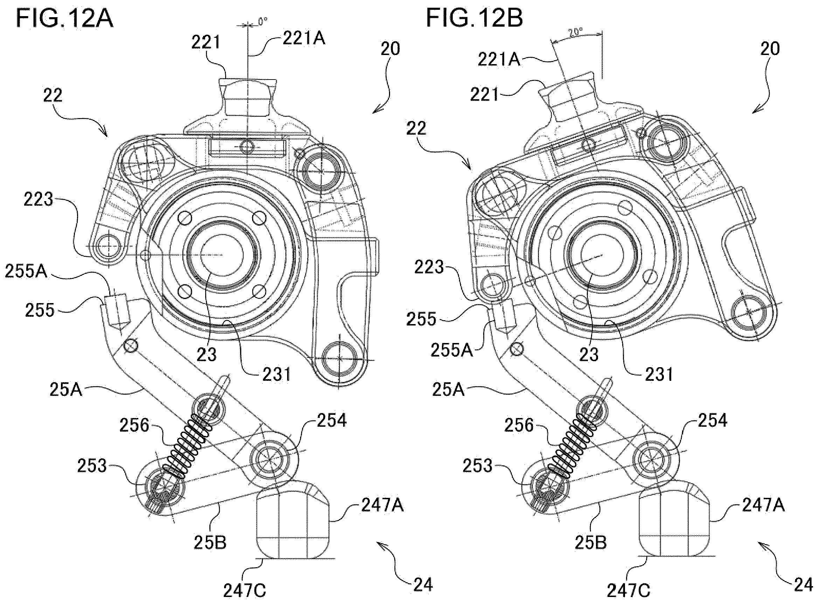

Figs. 12A and 12B illustrate the thigh connection portion 22 and the knee angle restrictor 24 for illustrating the rotational operation around the knee shaft 23, that is, the bending and stretching motion of the knee joint 20. Fig. 12A illustrates a state in which the knee angle θ, at which an axis 221A of the thigh attachment portion 221 of the thigh connection portion 22 and an axis of the lower leg portion 21 (not illustrated) are on a straight line (on a vertical line in Fig. 12A), is zero degrees. Fig. 12B illustrates a state in which a direction of the axis of the lower leg portion 21 is maintained (vertical direction in Fig. 12B), and the knee angle θ obtained by bending the axis 221A of the thigh attachment portion 221 of the thigh connection portion 22 by 20 degrees in the counterclockwise direction is 20 degrees.

The knee angle restrictor 24 is fixed to the lower leg portion 21 (not illustrated), and restricts the relative rotation of the thigh connection portion 22 and the lower leg portion 21 such that the knee angle θ formed by the thigh connection portion 22 with respect to the lower leg portion 21 does not exceed a predetermined lock angle θ_{L}. In the illustrated example, the lock angle θ_{L} is 20 degrees. Therefore, in the locked state in which the knee angle restrictor 24 restricts the knee angle θ to the lock angle θ_{L} or less, a value obtained between zero degrees in Fig. 12A and 20 degrees in Fig. 12B can be acquired as the knee angle θ, but a value outside this range cannot be acquired.

In Fig. 12B in which the knee angle θ is the maximum (lock angle θ_{L} = 20 degrees), a bending-side contact portion 223 provided on the bending side (counterclockwise side in Fig. 12B) on the outer periphery of the thigh connection portion 22 is stopped by coming into contact with the upper end portion 255 of the first link 25A. The bending-side contact portion 223 as a rotating member that rotates integrally with the thigh connection portion 22 is formed as an end surface of the thigh connection portion 22 that advances in the counterclockwise direction according to the bending motion of the knee joint 20, and comes into surface contact with the upper end portion 255 of the first link 25A on the path when the knee angle θ is the lock angle θ_{L} = 20 degrees. Since an elastic member 255A formed of an elastic material is provided on the contact surface of the upper end portion 255 of the first link 25A, the impact at the time of contact with the bending-side contact portion 223 can be alleviated. In the state of Fig. 12B in which the bending-side contact portion 223 and the upper end portion 255 of the first link 25A come into surface contact with each other, the thigh connection portion 22 is stopped by the upper end portion 255 of the first link 25A and cannot rotate in the counterclockwise direction (bending direction) any more. Therefore, the knee angle θ is restricted not to be greater than 20 degrees in Fig. 12B.

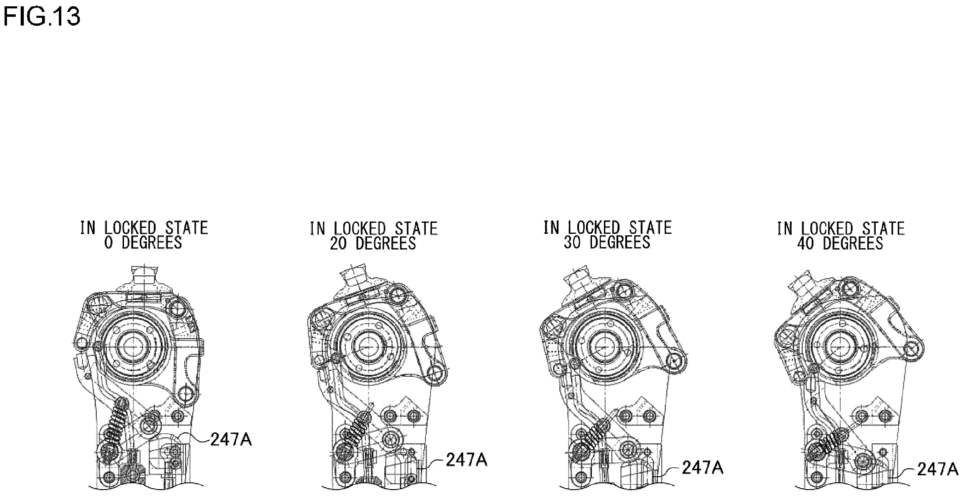

In Figs. 12A to 12B, the link connection portion 254 of the first link 25A and the second link 25B is always in contact with an elastic member 247A formed of an elastic material in the lower leg portion 21. Therefore, as compared with the elastic member 245A that comes into contact with the link connection portion 254 only at the lock angle (Fig. 9B) illustrated in Figs. 9A and 9B, the elastic member 247A can effectively alleviate vibration and impact caused by the bending and stretching motion of the knee joint 20 in the entire angle range in which the knee angle is equal to or less than the lock angle. Note that as illustrated in Fig. 11, an elastic member attaching and detaching portion 247B, to and from which the elastic member 247A can be attached and detached, is provided. The elastic member attaching and detaching portion 247B is configured by a screw of which a distal end comes into contact with a lower surface of a mount 247C that supports the elastic member 247A from below, and the position adjustment and the attachment and detachment of the elastic member 247A in the vertical direction in Fig. 11 can be performed by turning the screw. As described above, the elastic member attaching and detaching portion 247B functions as a position change portion for changing the position of the elastic member 247A and an elastic member replacement portion for replacing the elastic member 247A. As illustrated in Fig. 12B, since the position of the elastic member 247A determines the lock angle θ_{L}, the elastic member attaching and detaching portion 247B can change the lock angle θ_{L} according to the wearer of the knee joint 20. Fig. 13 illustrates four lock angles θ_{L} (0 degrees, 20 degrees, 30 degrees, 40 degrees in order from the left side) that differ depending on the position of the elastic member 247A.

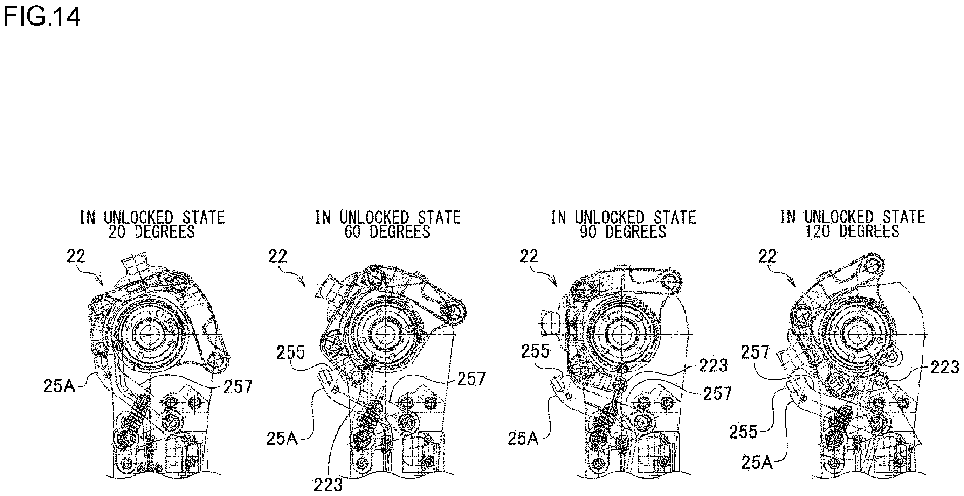

Fig. 14 illustrates the rotational operation of the thigh connection portion 22 in the unlocked state in which the knee angle restrictor 24 does not restrict the rotation of the knee shaft 23. The state in which the knee angle θ is 20 degrees, 60 degrees, 90 degrees, and 120 degrees is illustrated in order from the left side. In a case where the knee angle θ exceeds the lock angle θ_{L} = 20 degrees, the first link 25A is pulled in a radial direction away from the knee shaft 23 by a mechanical switching means such as a lever or a wire (not illustrated), and thus the upper end portion 255 of the first link 25A deviates from the course of the bending-side contact portion 223 of the thigh connection portion 22. As a result, the state is switched to the unlocked state in which the thigh connection portion 22 can rotate in the counterclockwise direction (bending direction) beyond the lock angle θ_{L}. In the bending and stretching motion beyond the lock angle θ_{L} = 20 degrees, a guide surface 257 facing the thigh connection portion 22 performs guiding while making contact with the outer peripheral surface of the thigh connection portion 22 in the first link 25A, and thus the thigh connection portion 22 can smoothly rotate.

In contrast to JP H09-75382 A in which the knee joint 20 can be freely bent in a state in which the knee angle θ is equal to or less than the lock angle θ_{L}, in the present configuration example, the resistance is applied to the rotation of the knee shaft 23 at the time of the bending motion by accommodating the rotational resistor or the rotational resistance module in the accommodation space 231 even in a state in which the knee angle θ is equal to or less than the lock angle θ_{L}. Due to the resistance applied by the rotational resistor or the rotational resistance module, for example, it is possible to prevent the sudden knee bending having the lock angle θ_{L} (Fig. 12B) from occurring from the state in which the knee angle θ, at which the axis 221A of the thigh attachment portion 221 of the thigh connection portion 22 and the axis of the lower leg portion 21 are on a straight line, is zero degrees (Fig. 12A). Therefore, according to the present configuration example, it is possible to reduce the falling down risk and anxiety of the wearer of the knee joint 20, which are caused by the sudden knee bending.

As described above, the present invention has been described based on the embodiment. It is understood by those skilled in the art that the embodiment is an example, various modifications can be made to each constituent element or a combination of processing processes, and such modifications are also within the scope of the technique of the present invention.

In the embodiment, various knee joints 20 including the thigh connection portion 22 and the lower leg portion 21 have been described. However, as long as the knee joint 20 operates normally, each constituent element of the knee joint 20 may be provided in either the thigh connection portion 22 or the lower leg portion 21, or may be provided outside the thigh connection portion 22 and the lower leg portion 21. That is, in the embodiment, the constituent elements provided in the thigh connection portion 22 may be provided in the lower leg portion 21 or outside, and in the embodiment, the constituent elements provided in the lower leg portion 21 may be provided in the thigh connection portion 22 or outside. For example, in the knee joint 20 illustrated in Figs. 6 to 13, the knee angle restrictor 24 for realizing the locked state, which is provided in the lower leg portion 21, may be provided in the thigh connection portion 22 by using the same mechanism as in each drawing.

A functional configuration of each device described in the embodiment can be realized by hardware resources or software resources, or by cooperation of the hardware resources and the software resources. A processor, a ROM, a RAM, and other LSIs can be used as the hardware resources. Programs such as an operating system and an application can be used as the software resources.

In the embodiment disclosed in the present specification, in providing a plurality of functions in a distributed manner, some or all of a plurality of the functions may be integrated, and conversely, in providing a plurality of the functions in the integrating manner, some or all of the plurality of functions can be distributed. The present invention may be configured to achieve the object of the invention regardless of whether the functions are integrated or distributed.

The present invention may be described by the following clauses:
Clause 21. A prosthetic leg (10) comprising:
   a thigh portion;
   a thigh connection portion (22) that is provided on the thigh portion side;
   a lower leg portion (21) that is connected to the thigh connection portion (22) and rotatably provided around a knee shaft;
   a knee angle restrictor (24) structured to restrict a relative rotation of the thigh connection portion (22) and
   the lower leg portion (21) such that a knee angle formed by the thigh connection portion (22) with respect to the lower leg portion (21) does not exceed a predetermined lock angle; and
   a rotational resistor structured to apply resistance to the rotation in a case where the knee angle is equal to or less than the lock angle.
Clause 22. A rotational resistance module which is capable of being attached to and detached from a knee joint (20) including: a thigh connection portion (22) that is provided on a thigh portion side; a lower leg portion (21) that is connected to the thigh connection portion (22) and rotatably provided around a knee shaft; and a knee angle restrictor (24) structured to restrict a relative rotation of the thigh connection portion (22) and the lower leg portion (21) such that a knee angle formed by the thigh connection portion (22) with respect to the lower leg portion (21) does not exceed a predetermined lock angle, wherein resistance is applied to the rotation in a case where the knee angle is equal to or less than the lock angle.
Clause 23. A knee joint (20) comprising:
   a thigh connection portion (22) that is provided on a thigh portion side;
   a lower leg portion (21) that is connected to the thigh connection portion (22) and rotatably provided around a knee shaft;
   a link mechanism in which one ends of a first link (25A) having the other end provided on the thigh connection portion (22) side and a second link (25B) having the other end provided on the lower leg portion (21) side are rotatably connected to each other; and
   a knee angle restrictor (24) structured to restrict a relative rotation of the thigh connection portion (22) and the lower leg portion (21) such that a knee angle formed by the thigh connection portion (22) with respect to the lower leg portion (21) does not exceed a predetermined lock angle, the knee angle restrictor (24) including an elastic member (245A) with which at least one of the first link (25A) or the second link (25B) comes into contact when at least the knee angle is the lock angle.
Clause 24. The knee joint (20) according to cluse 23, further comprising a position change portion (245B) for changing a position of the elastic member (245A).
Clause 25. The knee joint (20) according to clause 23 or 24, further comprising an elastic member (245A) replacement portion for replacing the elastic member (245A).

## Claims

1. A knee joint (20) comprising:
a thigh connection portion (22) that is provided on a thigh portion side;
a lower leg portion (21) that is connected to the thigh connection portion (22) and rotatably provided around a knee shaft;
a knee angle restrictor (24) structured to restrict a relative rotation of the thigh connection portion (22) and
the lower leg portion (21) such that a knee angle formed by the thigh connection portion (22) with respect to the lower leg portion (21) does not exceed a predetermined lock angle; and
a rotational resistor structured to apply resistance to the rotation in a case where the knee angle is equal to or less than the lock angle.

2. The knee joint (20) according to claim 1, wherein the rotational resistor applies the resistance to the rotation in an entire angle range in which the knee angle is equal to or less than the lock angle.

3. The knee joint (20) according to claim 2, wherein the rotational resistor applies the resistance to the rotation by using hydraulic pressure.

4. The knee joint (20) according to claim 2, wherein the rotational resistor applies the resistance to the rotation by using viscosity of a viscous fluid.

5. The knee joint (20) according to any one of claims 1 to 4, wherein a magnitude of the resistance applied to the rotation by the rotational resistor is constant regardless of the knee angle.

6. The knee joint (20) according to any one of claims 1 to 4, wherein the rotational resistor changes a magnitude of the resistance applied to the rotation.

7. The knee joint (20) according to claim 6, wherein the rotational resistor increases the resistance applied to the rotation according to an increase in the knee angle.

8. The knee joint (20) according to claim 6, wherein the rotational resistor decreases the resistance applied to the rotation according to an increase in the knee angle.

9. The knee joint (20) according to any one of claims 1 to 8, further comprising an angular velocity detector structured to detect an angular velocity of the knee angle,
wherein the rotational resistor changes the resistance applied to the rotation according to the angular velocity detected by the angular velocity detector.

10. The knee joint (20) according to claim 9, wherein the rotational resistor increases the resistance applied to the rotation according to an increase in the angular velocity.

11. The knee joint (20) according to claim 9, wherein the rotational resistor decreases the resistance applied to the rotation according to an increase in the angular velocity.

12. The knee joint (20) according to any one of claims 1 to 11, further comprising a link mechanism in which one ends of a first link (25A) having the other end provided on the thigh connection portion (22) side and a second link (25B) having the other end provided on the lower leg portion (21) side are rotatably connected to each other,
wherein the knee angle restrictor (24) includes a stopping member (245) with which at least one of the first link (25A) or the second link (25B) comes into contact when at least the knee angle is the lock angle.

13. The knee joint (20) according to claim 12, wherein the stopping member (245) comes into contact with a connection portion of the first link (25A) and the second link (25B) when at least the knee angle is the lock angle.

14. The knee joint (20) according to claim 12 or 13, wherein the stopping member (245) is an elastic member (245A) having elasticity.

15. The knee joint (20) according to any one of claims 12 to 14, further comprising a position change portion (245B) for changing a position of the stopping member (245).

16. The knee joint (20) according to any one of claims 12 to 15, wherein one end of the second link (25B) is fixed to the lower leg portion (21), and
one end of the first link (25A) is movable with respect to the thigh connection portion (22) and is stopped by coming into contact with the thigh connection portion (22) when the knee angle is the lock angle.

17. The knee joint (20) according to any one of claims 12 to 16, further comprising a spring (256) that has one end connected to the first link (25A) and the other end connected to the second link (25B), and biases the first link (25A) and the second link (25B) in a direction in which the knee angle decreases.

18. The knee joint (20) according to any one of claims 1 to 11, wherein the knee angle restrictor (24) includes:
a rotating member that is provided in the thigh connection portion (22) and rotates integrally with the thigh connection portion (22); and
a stopping member (245) that is provided in the lower leg portion (21), and with which the rotating member comes into contact when the knee angle is the lock angle.

19. The knee joint (20) according to any one of claims 1 to 18, further comprising a mechanical switcher structured to mechanically perform switching between a locked state in which the knee angle restrictor (24) restricts the rotation and an unlocked state in which the knee angle restrictor (24) does not restrict the rotation.

20. The knee joint (20) according to any one of claims 1 to 18, further comprising an electrical switcher structured to electrically perform switching between a locked state in which the knee angle restrictor (24) restricts the rotation and an unlocked state in which the knee angle restrictor (24) does not restrict the rotation.
